# EUROPEAN PATENT SPECIFICATION

(11) **EP 3 661 471 B1**
(45) Date of publication and mention of the grant of the patent: **29.11.2023**
(21) Application number: 17920020.9
(22) Date of filing: 31.07.2017
(51) Int. Cl.: A61F 13/15, A61F 13/49

(54) **ELASTICIZED ABSORBENT ARTICLES AND METHODS OF FORMING ELASTICIZED ABSORBENT ARTICLES**
ELASTIFIZIERTE ABSORBIERENDE ARTIKEL UND VERFAHREN ZUR HERSTELLUNG ELASTIFIZIERTER ABSORBIERENDER ARTIKEL
ARTICLES ABSORBANTS ÉLASTIQUES ET PROCÉDÉS DE FORMATION D'ARTICLES ABSORBANTS ÉLASTIQUES

(43) Date of publication of application: 10.06.2020
(73) Proprietor: Kimberly-Clark Worldwide, Inc., Neenah, Wisconsin 54956 (US)
(72) Inventor: COENEN, Joseph, D., Neenah, Wisconsin 54956 (US); HAMEISTER, Jerry, L., Neenah, Wisconsin 54956 (US); RHODES, Brian, K., Neenah, Wisconsin 54956 (US)
(74) Representative: Dehns
(86) International application number: PCT/US2017/044660
(87) International publication number: WO 2019/027418

(56) References cited:
- KR-A- 20160 132 847
- US-A1- 2004 108 043
- US-A1- 2005 148 965
- US-A1- 2006 142 728
- US-A1- 2013 255 864
- US-A1- 2015 173 957
- US-A1- 2017 079 852

## Description

### TECHNICAL FIELD

The present disclosure is directed to elasticized absorbent articles, and more particularly to adhesive application patterns for construction of elasticized absorbent articles.

### BACKGROUND OF THE DISCLOSURE

One of the primary functions of personal care absorbent articles is to retain and absorb body exudates such as urine, fecal material, blood, and menses. Different varieties of disposable absorbent articles have different mechanisms for being retained on a wearer. For example, open diapers may have one or more Velcro-like attachment means for securing a rear portion of the diaper to the front portion of the diaper around the wearer's waist. Other absorbent articles such as diaper pants or adult pants may have a front waist panel that is permanently secured to a rear waist panel, with elastic strands running around the waist opening. Such absorbent articles are designed to be pulled on, with the elastic strands used to securely retain the article around the waist of the wearer. Both open diapers and absorbent pants may also have elastic strands running along the leg openings of the articles in order to secure the article around the legs of wearer.

Absorbent articles may be made in a variety of different manners. One group of general manufacturing processes are known as cross-direction (CD) processes. In CD processes, each of the front and rear waist panels travel during manufacturing in the machine direction, while the absorbent core is applied between the waist panels in the cross-machine direction. One of the beneficial features of these types of manufacturing processes is the ease of application of the elastic strands to the front and rear waist panels and along the leg openings, as the elastic strands may be applied in the direction of travel of the waist panel webs. There is a continued desire for improvements to CD manufacturing processes which reduce costs, increase production rates, and/or minimize waste.

US2015/0173957 A1 describes methods and apparatuses for making absorbent articles having contoured belts.

US2013/0255864 A1 describes apparatuses and methods for making absorbent articles.

### SUMMARY OF THE DISCLOSURE

The disclosure is directed to several alternative designs, materials, and methods of manufacturing absorbent articles. The invention defines a method for assembling an absorbent article in accordance with claim 1, and an absorbent article in accordance with claim 7.

Insofar as the term example(s) or embodiment(s) is used in the following or features are presented as being optional, this should be interpreted in such a way that only protection sought is that of the invention claimed. Reference(s) to "example(s)" or "embodiment(s)" throughout the description which are not under the scope of the appended claims merely represent possible exemplary executions and are not part of the present invention.

In a first illustrative example, a method for assembling an absorbent article, the absorbent article comprising a chassis comprising a front waist region having a front waist edge, a rear waist region having a rear waist edge, and a crotch region disposed between the front waist region and the rear waist region, and an absorbent insert disposed in the crotch region and coupled to the front waist region tad the rear waist region, may comprise forming an elasticated first waist panel material. Forming the elasticated first waist panel material may comprise advancing a first continuous substrate material having an upper surface and a lower surface in a machine direction, advancing a second continuous substrate material having an upper surface and a lower surface in the machine direction, advancing a plurality of elastomeric strands in a stretched state in the machine direction, applying a first adhesive intermittently to at least one of the plurality of elastomeric strands, applying a second adhesive intermittently to at least one of the lower surface of the first continuous substrate material and the upper surface of the second continuous substrate material, and placing the plurality of elastomeric strands between the lower surface of the first continuous substrate material and the upper surface of the second continuous substrate material forming the elasticated first waist panel material. The strands may be placed such that the elasticated first waist panel material comprises first adhesive regions comprising the first continuous substrate material, the second continuous substrate material, at least one of the plurality of elastomeric strands, and the intermittently applied first adhesive but not the intermittently applied second adhesive, and the elasticated first waist panel material comprises second adhesive regions comprising the first continuous substrate material, the second continuous substrate material, at least one of the plurality of elastomeric strands, and the intermittently applied second adhesive but not the intermittently applied first adhesive. The method may further comprise forming an elasticated second waist panel material, coupling an absorbent insert to the elasticated first waist panel material and the elasticated second waist panel material, the absorbent insert comprising an absorbent body and a bodyside liner, and severing the elasticated first waist panel material and the elasticated second waist panel material to form an individual absorbent article, the elasticated first waist panel material forming the front waist region and the elasticated second waist panel material forming the rear waist region.

In a second illustrative example, the method of the first illustrative example may further comprise bonding the elasticated first waist panel material to the elasticated second waist panel material.

In a third illustrative example, the method of any of the first and second illustrative examples may further comprise applying the first adhesive continuously to at least one of the plurality of elastomeric strands, and the elasticated first waist panel material may further comprise third adhesive regions comprising the first continuous substrate material, the second continuous substrate material, at least one of the plurality of elastomeric strands, and the continuously applied first adhesive but not the second adhesive.

In a fourth illustrative example, the at least one of the plurality of elastomeric strands of the third illustrative example having the first adhesive continuously applied may form at least a portion of a front waistband of the absorbent article and is located closer to the front waist edge of the absorbent article than any of the plurality of elastomeric strands having the first adhesive intermittently applied.

In a fifth illustrative example, the absorbent insert of any of the first through fourth illustrative examples may be coupled to the elasticated first waist panel material such that the absorbent body overlaps the second adhesive region and none of the first adhesive regions.

In a sixth illustrative example, the absorbent insert of the third illustrative example may be coupled to the elasticated first waist panel material such that the absorbent body overlaps the second adhesive region but does not overlap the third adhesive region.

In a seventh illustrative example, the first adhesive of any of the first through sixth illustrative examples may be an elastic adhesive.

In an eighth illustrative example, the second adhesive of any of the first through seventh illustrative examples may be a laminating adhesive.

In a ninth illustrative example, forming the elasticated second waist panel material of any of the first through eighth illustrative examples may comprise the steps of forming the elasticated first waist panel material.

In a tenth illustrative example, a method for assembling an absorbent article, the absorbent article comprising a chassis comprising a front waist region having a front waist edge, a rear waist region having a rear waist edge, and a crotch region disposed between the front waist region and the rear waist region, and an absorbent insert disposed in the crotch region and spanning from the front waist region to the rear waist region may comprise forming an elasticated front waist panel. Forming the elasticated front waist panel may comprise applying a first adhesive intermittently to a first one of a first front waist panel material, a second front waist panel material, and at least one of a plurality of front waist panel elastomeric strands, applying a second adhesive intermittently to a second, different one of the first front waist panel material, the second front waist panel material, and the at least one of the plurality of front waist panel elastomeric strands, and combining the first front waist panel material and the second front waist panel material with the at least one of the plurality of front waist panel elastomeric strands disposed therebetween forming first front panel adhesive regions where only the first adhesive is present and second front panel adhesive regions where only the second adhesive is present. The method may further comprise forming an elasticated rear waist panel which in turn may comprise applying the first adhesive intermittently to a first one of a first rear waist panel material, a second rear waist panel material, and at least one of a plurality of rear waist panel elastomeric strands, applying the second adhesive intermittently to a second, different one of the first rear waist panel material, the second rear waist panel material, and the at least one of the plurality of rear waist panel elastomeric strands, and combining the first rear waist panel material and the second rear waist panel material with the at least one of the plurality of rear waist panel elastomeric strands disposed therebetween forming first rear panel adhesive regions where only the first adhesive is present and second rear panel adhesive regions where only the second adhesive is present. The method of the tenth illustrative example may still further comprise coupling an absorbent insert to the elasticated front waist panel material and the elasticated rear waist panel material, the absorbent insert comprising an absorbent body and a bodyside liner and separating a portion of the elasticated front waist panel and the elasticated rear waist panel with the absorbent insert disposed therebetween to form an individual absorbent article.

In an eleventh illustrative example, the absorbent insert of the tenth illustrative example may be coupled to the elasticated front waist panel such that the absorbent body overlaps the second front panel adhesive region and the second rear panel adhesive region.

In a twelfth illustrative example, the method of any of the tenth and eleventh illustrative examples may further comprise applying one of the first adhesive and the second adhesive continuously to at least one of the front waist panel waist elastomeric strands.

In a thirteenth illustrative example, the method of any of the tenth through twelfth illustrative examples may further comprise severing at least one of the front waist panel elastomeric strands at a location within the second adhesive region of the front waist panel material and severing at least one of the rear waist panel elastomeric strands at a location within the second adhesive region of the rear waist pane material.

In a fourteenth illustrative example, the second front waist panel material and the first front waist panel material of any of the tenth through thirteenth illustrative examples may comprise the same web of material.

In a fifteenth illustrative example, the method of any of the tenth through fourteenth illustrative examples may further comprise bonding the elasticated front waist panel to the elasticated rear waist panel.

In a sixteenth illustrative examples, the first adhesive of any of the tenth through fifteenth illustrative examples may be different than the second adhesive.

In a seventeenth illustrative example, the first adhesive of any of the tenth through sixteenth illustrative examples may be applied intermittently to at least one of the front waist panel elastomeric strands and the rear waist panel elastomeric strands, and the first adhesive may be an elastic adhesive.

In an eighteenth illustrative example, an absorbent article may comprise a chassis comprising an elasticated front waist panel, the elasticated front waist panel comprising a front panel garment facing web, a front panel body facing web, and a plurality of elastomeric strands disposed between the front panel garment facing web and the front panel body facing web, the front waist panel further comprising a first front panel adhesive region comprising a first adhesive but not a second adhesive and a second front panel adhesive region comprising the second adhesive but not the first adhesive. The chassis may further comprise an elasticated rear waist panel, the elasticated rear waist panel comprising a rear panel garment facing web, a rear panel body facing web, and a plurality of elastomeric strands disposed between the rear panel garment facing web and the rear panel body facing web, the rear waist panel further comprising a first rear panel adhesive region comprising the first adhesive but not the second adhesive and a second rear panel adhesive region comprising the second adhesive but not the first adhesive and an absorbent insert coupled to the elasticated front waist panel and the elasticated rear waist panel, the absorbent insert comprising an absorbent body and a body facing liner.

In a nineteenth illustrative example, the absorbent article of the eighteenth illustrative example may comprise a single web that extends between the front waist panel and the rear waist panel forming the front panel garment facing web and the rear panel garment facing web.

In a twentieth illustrative example, the absorbent body of the absorbent insert of any of the eighteenth and nineteenth illustrative examples may overlap the second front panel adhesive region but not the first front panel adhesive region and the second rear panel adhesive region but not the first rear panel adhesive region.

The above summary of some example embodiments is not intended to describe each disclosed embodiment or every implementation of aspects of the disclosure.

### BRIEF DESCRIPTION OF DRAWINGS

The aspects of the disclosure may be further understood in consideration of the following detailed description of various embodiments in connection with the accompanying drawings, in which:
FIGS. 1A and 1B are perspective views of views of alternative systems 100A, 100B for forming an absorbent article, according to aspects of the present disclosure;
FIG. 2 is a close-up plan view of region 2 of FIG. 1A depicting adhesives and adhesive zones, according to aspects of the present disclosure;
FIG. 3 is a plan view of an exemplary absorbent article of the present disclosure in an un-folded and laid-flat configuration;
FIG. 4A is a plan view of the exemplary absorbent article of FIG. 3 with portions removed to depict adhesive and adhesive regions of the absorbent article;
FIG. 4B is another plan view of the exemplary absorbent article of FIG. 3 with portions removed to depict adhesive and adhesive regions of the absorbent article; and
FIG. 5 is a plan view of an alternative exemplary absorbent article, according to aspects of the present disclosure.

Repeat use of reference characters in the present specification and drawings is intended to represent the same or analogous features or elements of the disclosure. Additionally, while the aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the scope of the disclosure. The scope of the present invention is only intended to be limited by the appended claims.

### DETAILED DESCRIPTION OF THE DISLOSURE

The present disclosure is generally directed towards elasticized absorbent articles including differing, non-overlapping adhesive regions. In some processes for forming absorbent articles, differing adhesives may be applied to elastic strands and to one or more facing webs. The adhesive applied to the elastic strands, for example an elastic adhesive, may help maintain the position of the elastic strands within the absorbent article, while the adhesive applied to the one or more facing webs, for example a laminating adhesive, may prevent two webs bonded together from de-laminating. However, in the regions of the article where the elastic strands are present, and where the elastic adhesive is present, the elastic adhesive may operate to prevent the two webs sandwiching the elastic strands from de-laminating. Accordingly, the laminating adhesive may be redundant in these regions. In order to reduce costs, it may be beneficial to ensure that absorbent articles contain first regions where only the elastic adhesive is present and second regions where only the laminating adhesive is present.

The following detailed description should be read with reference to the drawings in which similar elements in different drawings are numbered the same. The detailed description and the drawings, which are not necessarily to scale, depict illustrative embodiments and are not intended to limit the scope of the disclosure. The illustrative embodiments depicted are intended only as exemplary. Selected features of any illustrative embodiment may be incorporated into an additional embodiment unless clearly stated to the contrary.

When introducing elements of the present disclosure or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements. Many modifications and variations of the present disclosure can be made without departing from the spirit and scope thereof. Therefore, the exemplary embodiments described above should not be used to limit the scope of the invention.

### Definitions:

The term "absorbent article" refers herein to an article which may be placed against or in proximity to the body (i.e., contiguous with the body) of the wearer to absorb and contain various liquid, solid, and semi-solid exudates discharged from the body. Such absorbent articles, as described herein, are intended to be discarded after a limited period of use instead of being laundered or otherwise restored for reuse. It is to be understood that the present disclosure is applicable to various disposable absorbent articles, including, but not limited to, diapers, training pants, youth pants, swim pants, feminine hygiene products, including, but not limited to, menstrual pads, incontinence products, medical garments, surgical pads and bandages, other personal care or health care garments, and the like without departing from the scope of the present disclosure.

The term "bonded" refers herein to the joining, adhering, connecting, attaching, or the like, of two elements. Two elements will be considered bonded together when they are joined, adhered, connected, attached, or the like, directly to one another or indirectly to one another, such as when each is directly bonded to intermediate elements. The bonding of one element to another can occur via continuous or intermittent bonds.

The term "carded web" refers herein to a web containing natural or synthetic staple length fibers typically having fiber lengths less than about 100 mm. Bales of staple fibers can undergo an opening process to separate the fibers which are then sent to a carding process which separates and combs the fibers to align them in the machine direction after which the fibers are deposited onto a moving wire for further processing. Such webs are usually subjected to some type of bonding process such as thermal bonding using heat and/or pressure. In addition to or in lieu thereof, the fibers may be subject to adhesive processes to bind the fibers together such as by the use of powder adhesives. The carded web may be subjected to fluid entangling, such as hydroentangling, to further intertwine the fibers and thereby improve the integrity of the carded web. Carded webs, due to the fiber alignment in the machine direction, once bonded, will typically have more machine direction strength than cross machine direction strength.

The term "film" refers herein to a thermoplastic film made using an extrusion and/or forming process, such as a cast film or blown film extrusion process. The term includes apertured films, slit films, and other porous films which constitute liquid transfer films, as well as films which do not transfer fluids, such as, but not limited to, barrier films, filled films, breathable films, and oriented films.

The term "gsm" refers herein to grams per square meter.

The term "hydrophilic" refers herein to fibers or the surfaces of fibers which are wetted by aqueous liquids in contact with the fibers. The degree of wetting of the materials can, in turn, be described in terms of the contact angles and the surface tensions of the liquids and materials involved. Equipment and techniques suitable for measuring the wettability of particular fiber materials or blends of fiber materials can be provided by Cahn SFA-222 Surface Force Analyzer System, or a substantially equivalent system. When measured with this system, fibers having contact angles less than 90 are designated "wettable" or hydrophilic, and fibers having contact angles greater than 90 are designated "nonwettable" or hydrophobic.

The term "liquid impermeable" refers herein to a layer or multi-layer laminate in which liquid body exudates, such as urine, will not pass through the layer or laminate, under ordinary use conditions, in a direction generally perpendicular to the plane of the layer or laminate at the point of liquid contact.

The term "liquid permeable" refers herein to any material that is not liquid impermeable.

The term "meltblown" refers herein to fibers formed by extruding a molten thermoplastic material through a plurality of fine, usually circular, die capillaries as molten threads or filaments into converging high velocity heated gas (e.g., air) streams which attenuate the filaments of molten thermoplastic material to reduce their diameter, which can be a microfiber diameter. Thereafter, the meltblown fibers are carried by the high velocity gas stream and are deposited on a collecting surface to form a web of randomly dispersed meltblown fibers. Such a process is disclosed, for example, in U.S. Patent No. 3,849,241 to Butin et al., which is incorporated herein by reference. Meltblown fibers are microfibers which may be continuous or discontinuous, are generally smaller than about 0.6 denier, and may be tacky and self-bonding when deposited onto a collecting surface.

The term "nonwoven" refers herein to materials and webs of material which are formed without the aid of a textile weaving or knitting process. The materials and webs of materials can have a structure of individual fibers, filaments, or threads (collectively referred to as "fibers") which can be interlaid, but not in an identifiable manner as in a knitted fabric. Nonwoven materials or webs can be formed from many processes such as, but not limited to, meltblowing processes, spunbonding processes, carded web processes, etc.

The term "spunbond" refers herein to small diameter fibers which are formed by extruding molten thermoplastic material as filaments from a plurality of fine capillaries of a spinnerette having a circular or other configuration, with the diameter of the extruded filaments then being rapidly reduced by a conventional process such as, for example, eductive drawing, and processes that are described in U.S. Patent No. 4,340,563 to Appel et al., U.S. Patent No. 3,692,618 to Dorschner et al., U.S. Patent No. 3,802,817 to Matsuki et al., U.S. Patent Nos. 3,338,992 and 3,341,394 to Kinney, U.S. Patent No. 3,502,763 to Hartmann, U.S. Patent No. 3,502,538 to Peterson, and U.S. Patent No. 3,542,615 to Dobo et al., each of which is incorporated herein in its entirety by reference. Spunbond fibers are generally continuous and often have average deniers larger than about 0.3, and in an embodiment, between about 0.6, 5 and 10 and about 15, 20 and 40. Spunbond fibers are generally not tacky when they are deposited on a collecting surface.

The term "superabsorbent" refers herein to a water-swellable, water-insoluble organic or inorganic material capable, under the most favorable conditions, of absorbing at least about 15 times its weight and, in an embodiment, at least about 30 times its weight, in an aqueous solution containing 0.9 weight percent sodium chloride. The superabsorbent materials can be natural, synthetic and modified natural polymers and materials. In addition, the superabsorbent materials can be inorganic materials, such as silica gels, or organic compounds, such as cross-linked polymers.

The term "thermoplastic" refers herein to a material which softens and which can be shaped when exposed to heat and which substantially returns to a non-softened condition when cooled.

The term "user" or "caregiver" refers herein to one who fits an absorbent article, such as, but not limited to, a diaper, training pant, youth pant, incontinent product, or other absorbent article about the wearer of one of these absorbent articles. A user and a wearer can be one and the same person.

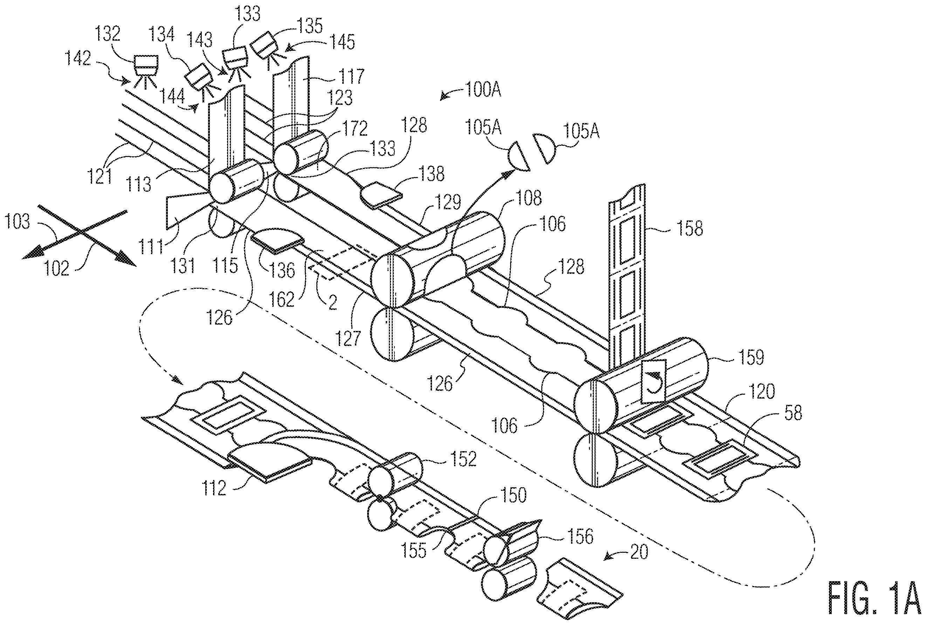

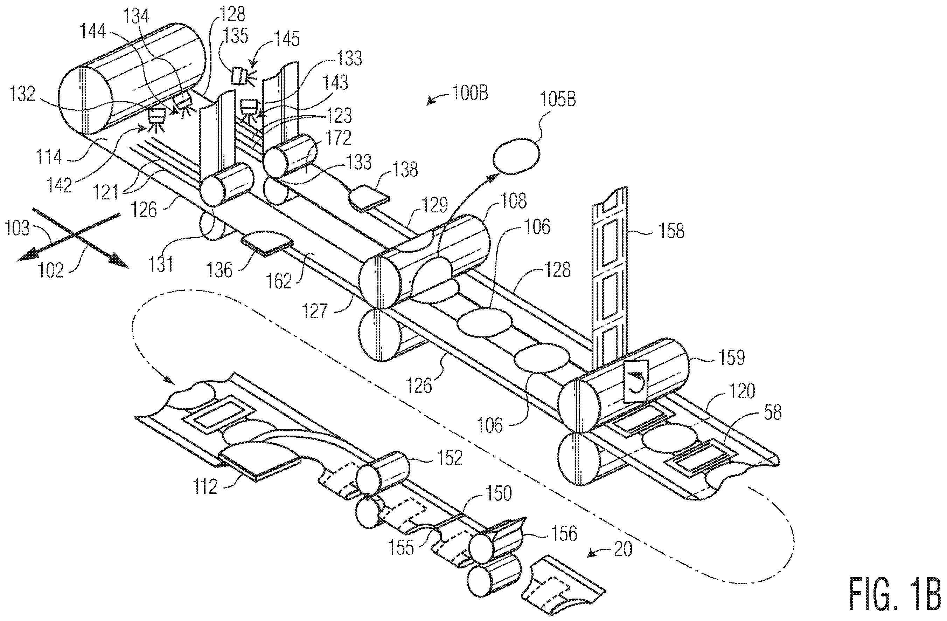

FIGS. 1A and 1B depict alternative methods for forming absorbent articles according the present disclosure: methods 100A and 100B, respectively. According to method 100A of FIG. 1A, an elasticated front waist panel 162 having a front waist edge 126 may be formed by advancing a first front waist panel web 111, a second front waist panel web 113, and one or more front waist panel elastomeric strands 121 in a machine direction 102. The one or more front waist panel elastomeric strands 121 are positioned between the first front waist panel web 111 and the second front waist panel web 113 and may be applied to one of the webs 111, 113. Likewise, an elasticated rear waist panel 172 having a rear waist edge 128 may be formed by advancing a first rear waist panel web 115, a second rear waist panel web 117, and one or more rear waist panel elastomeric strands 123 in the machine direction 102. The one or more rear waist panel elastomeric strands 123 are positioned between the first rear waist panel web 115 and the second rear waist panel web 117 and may be applied to one of the webs. Any of the webs 111, 113, 115, 117 may also be termed substrate materials throughout the present disclosure.

In order to facilitate bonding of the webs 111, 113 with the elastomeric strands 121 and the webs 115, 117 with the elastomeric strands 123, adhesive may be applied to the webs 111, 113, 115, 117 and/or the elastomeric strands 121, 123. In some embodiments a first adhesive 142 may be applied by adhesive applicator 132 to at least some of the one or more elastomeric strands 121. A second adhesive 144 may be applied by adhesive applicator 134 to web 113. Although, in other embodiments the adhesive applicator 134 may be positioned to apply the adhesive 144 to the web 111, or there may even multiple adhesive applicators 134 which apply the adhesive 144 to both webs 111 and 113. Of course, each of the webs 111, 113 have both body-facing surfaces and garment facing surfaces, with the body facing surfaces being the upward facing surfaces as viewed in FIGS. 1A and 1B. In embodiments where the adhesive 144 is applied to web 111, the adhesive may be applied to a body facing surface of the web 111, while if the adhesive 144 is applied to the web 1113, the adhesive 143 may be applied to the garment facing surface of web 113.

In any such embodiments, to form the front waist panel 162, the adhesive 144 may be applied in an intermittent fashion to webs 111 and/or 113. In the embodiments where the adhesive 144 is applied to both of webs 111, 113, the adhesive 144 may be applied intermittently to both webs 111, 1113 in a coordinated manner such that the adhesive 144 applied to both webs 111, 113 match-up when the webs 111, 113 are brought together at nip 131. Additionally, for the elastomeric strands 121 to which the adhesive 142 is applied, the adhesive 142 may also be applied in an intermittent manner. The intermittent application to both of the strands 121 and the webs 111 and/or 113 may be done in a coordinated fashion such that there is minimal or no overlap of the adhesive 142 with the adhesive 144 when the strands 121 and the webs 111, 113 are brought together.

For the rear waist panel 172, the first adhesive 143 and the second adhesive 145, applied by adhesive applicators 133, 135, respectively, may be applied to the strands 123 and the webs 115, 117 in the same manner as described with respect to the front waist panel 162. The webs 111, 113, with the strands 121 disposed therebetween, and the webs 115, 117 with the strands 123 disposed therebetween are then brought together at nips 131 and 133, respectively, where the webs 111, 113, 115, 117 and strands 121, 123 are bonded together forming the front and back waist panels 162, 172.

Forming the front and back waist panels 162, 172 in this manner will create first adhesive regions within the panels 162, 172 where the first adhesive142, 143 is present but the second adhesive 144, 145 is not present and second adhesive regions where the second adhesive 144, 145 is present and the first adhesive 142, 143 is not present. These adhesive regions will be described in more detail below with respect to other figures.

In at least some embodiments, the first adhesives 142, 143 may be the same adhesive. Some suitable adhesives for first adhesives 142, 143 are elastic adhesives. Such elastic adhesives are generally known in the art. The second adhesives 144, 145 may also be the same adhesive in some embodiments. Some suitable adhesives for second adhesives 144, 145 may comprise what are known as facing adhesives, or laminating adhesives, in the art. Accordingly, in embodiments in accordance with the claims, the first adhesives 142, 143 differ from the second adhesives 144, 145. However, in some examples not in accordance with the claims, all of the adhesives 142, 143, 144, and 145 may be the same adhesive.

The method 100A in particular embodiments further includes providing a supply 158 of individual absorbent inserts 58, superposing individual absorbent inserts 58 between the front waist panel 162 and the rear waist panel 172, and attaching the individual absorbent inserts 58 to the front waist panel162 and the rear waist panel 172. As will be shown in more detail, in at least some embodiments, the individual absorbent inserts 58 may be coupled to the front waist panel 162 and the rear waist panel 172 such that the individual absorbent inserts 58 overlap the second adhesive regions of the front waist panel 162 and the rear waist panel 172 but not the first adhesive regions of the front waist panel 162 and the rear waist panel 172. In certain embodiments, the absorbent inserts 58 may be manufactured in one orientation, as indicated by the supply 158 of individual absorbent inserts 58, and then cut and rotated 90 degrees (such as at cut-and-rotate station 159) before attachment to the front waist panel 162 and the rear waist panel 172.

The method can in particular embodiments further include removing portions 105A of the front waist panel 162 and the rear waist panel 172 (such as at cutting station 108) to define a series of spaced apart indentations 106. Such steps produce a definition in the front waist panel 162 and the rear waist panel 172 of an interconnected series 120 of disposable absorbent articles 20. Although the portions 105A are shown being removed prior to application of the individual absorbent inserts 58, it should be understood that such removal may happen at different locations within method 100A in different embodiments.

The method 100A can further include folding the interconnected series 120 of disposable absorbent articles 20, such as at an article folding station 112, along a transversely centered longitudinal fold line that extends in the machine direction 102, such that the front waist edge 126 is brought into close proximity with the back waist edge 128. In particular embodiments, the method 100A further comprises attaching the front waist panel 162 to the rear waist panel 172 to create a series of side seam bonds 150 (such as at seaming station 152) spaced apart in the machine direction 102. The method additionally comprises cutting the folded, interconnected series 120 of disposable absorbent articles 20 at a series of cut locations 155 (such as at cutting station 156) spaced apart in the machine direction 102 to create the plurality of disposable absorbent articles 20.

In further embodiments, the method 100A may include additional steps. For example, the method 100A may further include attaching a continuous leg elastic member (not shown) to one or both of the front waist panel 162 and the rear waist panel 172. In such embodiments, the leg elastic member(s) may extend in the machine direction 102 and overlapping the leg elastic member(s) with the front waist panel 162 and/or the rear waist panel 172 and positioning a carrier sheet (not shown) over the leg elastic member(s). The carrier sheet may then be bonded to the front waist panel 162 and/or the rear waist panel 172 with the leg elastic member(s) disposed between the carrier sheet and the front waist panel 162 and/or the rear waist panel 172. The leg elastic member(s) may further be severed in some embodiments at a series of elastic cut points spaced apart in the machine direction 102, each elastic cut point being generally aligned in the machine direction 102 with a respective individual absorbent assembly 58 (not shown).

Additionally or alternatively in still further embodiments, the method 100A may further include folding the front waist edge 126 of the first front waist panel web 111, such as at a front waistband folding station 136, to create a front waist edge fold 127 and to encase one or more of the front waist panel elastomeric strands 121 forming a front waistband region. In such an embodiment, the front waist edge fold 127 defines the front waist edge 126 of the article 20. The method may instead or additionally include folding the back waist edge 128 of the first rear waist panel web 115, such as at back waistband folding station 138, to create a back waist edge fold 129 and to encase one or more of the rear waist panel elastomeric strands 123 forming a rear waistband region. In such an embodiment, the back waist edge fold 129 defines the back waist edge 128 of the article 20.

Folding the webs 111 and/or 115 is only one possible method of forming waistband regions of the article 20. Other methods may include attaching a web material and folding the attached web material to encase one or more of the strands 121 and/or 123. Alternatively, the attached web may be an elasticized web, providing an elastic function without needing to encase one or more of the strands 121 and/or 123. In embodiments where one or more of the strands 121 and/or 123 are encased forming waistband region(s), the first adhesive 142, 143 may be applied to the strands 121 and/or 123 in a continuous fashion, in contrast to the intermittent application to one or more of the strands 121 and/or 123 which do not form part of any waistband regions. The various different adhesive patterns and regions is described in more detail below with respect to additional figures.

In still further additional or alternative embodiments, one or more of the elastomeric strands 121, 123 may be de-elasticized at one or more locations on the strands 121 and/or 123. For instance, the individual absorbent inserts 58 may be connected to the front waist panel 162 and/or the rear waist panel 172 overlapping one or more of the strands 121, 123. The elastomeric nature of the strands 121, 123 may cause an undesirable bunching of the webs 111, 113, 115, and/or 117 and/or of the individual absorbent inserts 58. Accordingly, the strands 121 and/or 123 may be de-elasticized in the region where the strands 121, 123 overlaps the individual absorbent inserts 58. One method of de-elasticizing the strands 121 and/or 123 is to sever the strands. In such embodiments, the strands 121 and/or 123 may be severed at one or more locations along their length in the machine direction 102 where the strands 121 and/or 123 overlap the individual absorbent inserts 58. As will be recognized, this region where the strands 121 and/or 123 overlap the individual absorbent inserts 58 correspond to the second regions 204 where only the second adhesive 144 is present. The second adhesive 144 may not be sufficiently strong enough to prevent the strands 121 and/or 123 from retracting after they have been severed. Accordingly, after the strands 121 and/or 123 have been severed, the strands 121 and/or 123 may retract toward the first regions 202.

FIG. 1B depicts alternative method 100B for forming the disposable absorbent articles 20. The method of FIG. 1B is very similar to the method of FIG. 1A, with a few key differences. One difference is that, instead of providing the first front waist panel web 111 and the first rear waist panel material 115, the method 100B uses a unitary first waist panel material 114. As can be seen in FIG. 1B, the unitary first waist panel material 114 has a width in the cross-machine direction 103 sufficient to span between and overlap both of the second front waist panel material 113 and the second rear waist panel material 117. Accordingly, the front waist panel 162 comprises the unitary first waist panel material 114, the one or more front waist panel elastomeric strands 121, and the second front waist panel material 113, while the rear waist panel 172 comprises the unitary first waist panel material 114, the one or more rear waist panel elastomeric strands 123, and the second rear waist panel material 117. Additionally, in such embodiments, unitary portions 105B may be removed at the cutting station 108 as opposed to the two separate portions 105A of the method 100A. The other additional, optional steps described with respect to the method 100A may be used in conjunction with the method 100B in a similar manner.

For clarity and ease of description, the further embodiments described in the present disclosure relate to disposable absorbent articles 20 formed according to the method 100A. It should be understood that this is not intended to limit the scope of this disclosure in any way.

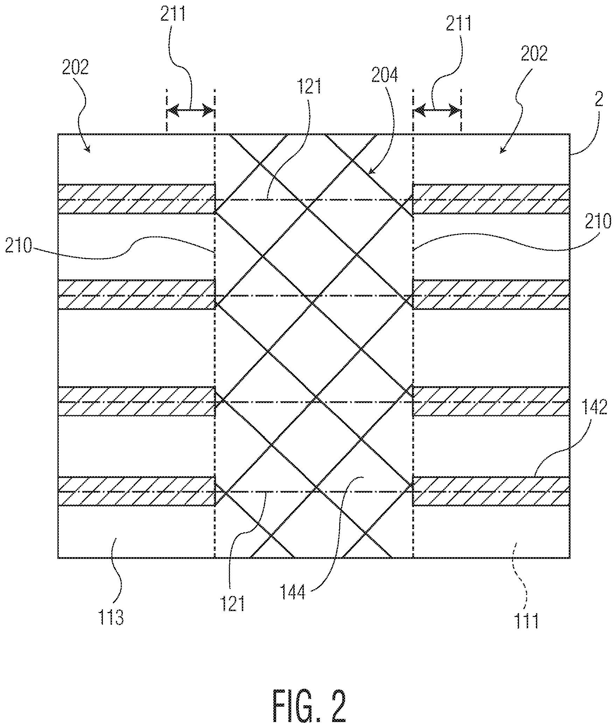

FIG. 2 is a close-up of box 2 of FIG. 1, depicting a region of the front waist panel 162 comprising the first front waist panel web 111, the second front waist panel web 113, and the one or more front waist panel elastomeric strands 121, and also depicting first adhesive regions 202 and a second adhesive region 204, formed by the first adhesive 142 and the second adhesive 144 that have been applied to the one or more front waist panel elastomeric strands 121 and to the first front waist panel web 111 and/or the second front waist panel web 113.

As can be seen in FIG. 2, neither of the first adhesive 142, which is depicted by angled hatching disposed around the front waist panel elastomeric strands 121 in the first regions 202, and the second adhesive 144, which is depicted by cross-hatching disposed in the second region 204 overlap. Lines 210 depict the boundaries between the first regions 202 and the second region 204. However, in some embodiments, the first adhesive 142 and the second adhesive 144 may partially overlap. For instance, there may be an overlap zone (not shown) proximate lines 210 where both the first adhesive 142 and the second adhesive 144 are present. In some embodiments, this overlap zone may have a zone width 211 which may have a value between about 1mm and about 20mm, or between about 2mm and about 10mm, or about 1mm, about 2mm, about 3mm, about 4mm, about 5mm, about 6mm, about 7mm, about 8mm, about 9mm, about 10mm, about 11mm, about 12mm, about 13mm, about 14mm, about 15mm, or about any other suitable value. Such overlap zones may occur where the adhesive applicators 132, 134 have a slow enough response time and/or the adhesives 142, 144 have viscosities low enough such that flow of the adhesives 142, 144 may not be able to be started and stopped sufficiently quickly to provide exacting, discrete regions 202, 204.

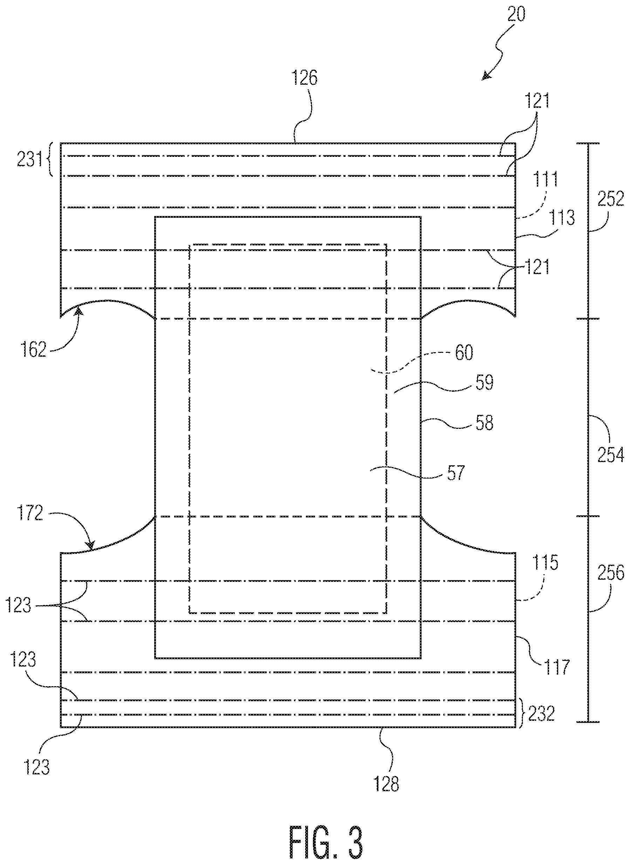

FIG. 3 is a plan view of an exemplary absorbent article 20 in an unfolded and laid-flat configuration. The article 20 has a front waist region 252, a crotch region 254, and a rear waist region 256 and may extend in a longitudinal direction between a front waist edge 126 and a rear waist edge 128. The front waist region 252 may be defined by the longitudinal extent of the second front waist panel web 113, while the rear waist region 256 may be defined by the longitudinal extent of the second rear waist panel web 117. In some embodiments, each of the regions 252, 254, and 256 may represent one third of an overall longitudinal length of the article 20, but this is not necessary in all embodiments.

The article 20 comprises front waist panel 162, rear waist panel 172, and absorbent insert 58 coupled to the front waist panel 162 and the rear waist panel 172. The absorbent insert 58 may generally comprise an outer cover 60, a bodyside liner 57, and an absorbent body 59 disposed between the outer cover 60 and the bodyside liner 57. In some embodiments, a lateral extent of the outer cover 60 and the bodyside liner 57 may be greater than the lateral extent of the absorbent body 59. In such embodiments, and the outer cover 60 and the bodyside liner 57 may be bonded together to fully enclose the absorbent body 59 on all sides. Although, in still other embodiments, the bodyside liner 57 may not extend beyond the absorbent body 59.

The bodyside liner 57 can be manufactured from a wide selection of materials, such as synthetic fibers (for example, polyester or polypropylene fibers), natural fibers (for example, wood or cotton fibers), a combination of natural and synthetic fibers, porous foams, reticulated foams, apertured plastic films, or the like. Examples of suitable materials include, but are not limited to, rayon, wood, cotton, polyester, polypropylene, polyethylene, nylon, or other heat-bondable fibers, polyolefins, such as, but not limited to, copolymers of polypropylene and polyethylene, linear low-density polyethylene, and aliphatic esters such as polylactic acid, finely perforated film webs, net materials, and the like, as well as combinations thereof.

Various woven and non-woven fabrics can be used for the bodyside liner 57. The bodyside liner 57 can include a woven fabric, a nonwoven fabric, a polymer film, a film-fabric laminate or the like, as well as combinations thereof. Examples of a nonwoven fabric can include spunbond fabric, meltblown fabric, coform fabric, carded web, bonded-carded web, bicomponent spunbond fabric, spunlace, or the like, as well as combinations thereof. The bodyside liner 57 need not be a unitary layer structure, and thus, can include more than one layer of fabrics, films, and/or webs, as well as combinations thereof. For example, the bodyside liner 57 can include a support layer and a projection layer that can be hydroentagled. The projection layer can include hollow projections, such as those disclosed in U.S. Patent Number 9,474,660, to Kirby, Scott S.C. et al, the entirety of which is herein incorporated by reference.

In further examples, the bodyside liner 57 can be composed of a meltblown or spunbond web of polyolefin fibers. Alternatively, the bodyside liner 57 can be a bonded-carded web composed of natural and/or synthetic fibers. The bodyside liner 57 can be composed of a substantially hydrophobic material, and the hydrophobic material can, optionally, be treated with a surfactant or otherwise processed to impart a desired level of wettability and hydrophilicity. The surfactant can be applied by any conventional means, such as spraying, printing, brush coating or the like. The surfactant can be applied to the entire bodyside liner 57 or it can be selectively applied to particular sections of the bodyside liner 57.

In an embodiment, a bodyside liner 57 can be constructed of a non-woven bicomponent web. The non-woven bicomponent web can be a spunbonded bicomponent web, or a bonded-carded bicomponent web. An example of a bicomponent staple fiber includes a polyethylene/polypropylene bicomponent fiber. In this particular bicomponent fiber, the polypropylene forms the core and the polyethylene forms the sheath of the fiber. Fibers having other orientations, such as multi-lobe, side-by-side, end-to-end may be used without departing from the scope of this disclosure. In an embodiment, a bodyside liner 57 can be a spunbond substrate with a basis weight from about 10 or 12 to about 15 or 20 gsm. In an embodiment, a bodyside liner 57 can be a 12 gsm spunbond-meltblown-spunbond substrate having 10% meltblown content applied between the two spunbond layers.

The outer cover 60 and/or portions thereof can be breathable and/or liquid impermeable. The outer cover 60 and/or portions thereof can be elastic, stretchable, or non-stretchable. The outer cover 60 may be constructed of a single layer, multiple layers, laminates, spunbond fabrics, films, meltblown fabrics, elastic netting, microporous webs, bonded-carded webs or foams provided by elastomeric or polymeric materials. In an embodiment, for example, the outer cover 60 can be constructed of a microporous polymeric film, such as polyethylene or polypropylene.

In some specific embodiments, the outer cover 60 can be a single layer of a liquid impermeable material, such as a polymeric film. In other embodiments, the outer cover 60 can be a multi-layered laminate in which at least one of the layers is liquid impermeable. In some embodiments, the outer cover 60 can be a two layer construction, including an outer layer (not shown) and an inner layer (not shown) which can be bonded together such as by a laminate adhesive. Suitable laminate adhesives can be applied continuously or intermittently as beads, a spray, parallel swirls, or the like, but it is to be understood that the inner layer can be bonded to the outer layer by other bonding methods, including, but not limited to, ultrasonic bonds, thermal bonds, pressure bonds, or the like.

The outer layer of the outer cover 60 can be any suitable material and may be one that provides a generally cloth-like texture or appearance to the wearer. An example of such material can be a 100% polypropylene bonded-carded web with a diamond bond pattern available from Sandler A.G., Germany, such as 30 gsm Sawabond 4185^{®} or equivalent. Another example of material suitable for use as an outer layer of an outer cover 60 can be a 20 gsm spunbond polypropylene non-woven web. The outer layer may also be constructed of the same materials from which the bodyside liner 60 can be constructed as described herein.

The liquid impermeable inner layer of the outer cover 60 (or the liquid impermeable outer cover 60 where the outer cover 60 is of a single-layer construction) can be either vapor permeable (i.e., "breathable") or vapor impermeable. The liquid impermeable inner layer (or the liquid impermeable outer cover 60 where the outer cover 60 is of a single-layer construction) can be manufactured from a thin plastic film. The liquid impermeable inner layer (or the liquid impermeable outer cover 60 where the outer cover 60 is of a single-layer construction) can inhibit liquid body exudates from leaking out of the absorbent article 20 and wetting articles, such as bed sheets and clothing, as well as the wearer and caregiver.

As can be seen, the front waist panel 162 may generally be located within the front waist region 252 while the rear waist panel 172 may generally be located within the rear waist region 256. Each of the panels 162, 172 are shown comprising a number of elastomeric strands 121, 123. Although in the embodiment of FIG. 3, each panel 162, 172 is shown comprising five (5) elastomeric strands 121, 123, this is not meant to be limiting in any way. For example, in alternative embodiments, each of the panels 162, 172 may comprise any suitable number of elastomeric strands 121, 123, such as between about 3 and about 20, or between about 5 and about 15, or between about 7 and about 13. Additionally, in some embodiments, the number of elastomeric strands 121, 123 may be different in each of the panels 121, 123.

The strands 121, 123 can be formed from rubber or other elastomeric materials. Some suitable materials include Lycra^{®} brand elastic filaments available from the DuPont Corporation. In some embodiments, the elastomeric strands 121, 123 are made of a Creora Spandex 940 decitex, which corresponds to a diameter of about 0.016 inches per strand. Additionally, each of the individual strands 121, 123 may be comprised of between about 10 and about 50 micro-strands that are wound together to form composite elastic strands.

Generally, the webs 111, 113, 115, and 117 may comprise any suitable material for use in an absorbent article, such as article 20. Webs 113 and 117 are body-facing webs, which may be in contact with a wearer when the article 20 is worn. Accordingly, the webs 113, 117 may comprise any of the materials described with respect to the bodyside liner 57. For instance, the webs 113, 117 may preferably have a soft feel for comfort when worn against a body. Webs 111, 115, in contrast, are garment facing webs which may be exposed to clothing garments when worn by a user. Generally, webs 111, 115 may also comprise any of the materials described with respect to the bodyside liner 57 and may be the same material as the corresponding webs 113, 117. Although, in other embodiments webs 111, 115 may comprise any of the materials described with respect to the outer cover 60. For instance, it may be beneficial in some embodiments for the webs 113, 117 to be liquid impermeable in order to perform a function similar to the outer cover 60 in preventing leakage of liquids out of the article 20 and onto the clothing garments of a wearer.

The absorbent body 59 can be suitably constructed to be generally compressible, conformable, pliable, non-irritating to the wearer's skin and capable of absorbing and retaining liquid body exudates. The absorbent body 59 can be manufactured in a wide variety of sizes and shapes (for example, rectangular, trapezoidal, T-shape, I-shape, hourglass shape, etc.) and from a wide variety of materials. The size and the absorbent capacity of the absorbent body 59 may generally be compatible with the size of the intended wearer (infants to adults) and the liquid loading imparted by the intended use of the absorbent article 20. The absorbent body 59 can have a length and width that can be less than or equal to the length and width of the absorbent article 20.

In an embodiment, the absorbent body 20 can be composed of a web material of hydrophilic fibers, cellulosic fibers (e.g., wood pulp fibers), natural fibers, synthetic fibers, woven or nonwoven sheets, scrim netting or other stabilizing structures, superabsorbent material, binder materials, surfactants, selected hydrophobic and hydrophilic materials, pigments, lotions, odor control agents or the like, as well as combinations thereof. In an embodiment, the absorbent body 59 can be a matrix of cellulosic fluff and superabsorbent material. In an embodiment, the absorbent body 59 may be constructed of a single layer of materials, or in the alternative, may be constructed of two or more layers of materials.

Various types of wettable, hydrophilic fibers can be used in the absorbent body 59. Examples of suitable fibers include natural fibers, cellulosic fibers, synthetic fibers composed of cellulose or cellulose derivatives, such as rayon fibers; inorganic fibers composed of an inherently wettable material, such as glass fibers; synthetic fibers made from inherently wettable thermoplastic polymers, such as particular polyester or polyamide fibers, or composed of nonwettable thermoplastic polymers, such as polyolefin fibers which have been hydrophilized by suitable means. The fibers may be hydrophilized, for example, by treatment with a surfactant, treatment with silica, treatment with a material which has a suitable hydrophilic moiety and is not readily removed from the fiber, or by sheathing the nonwettable, hydrophobic fiber with a hydrophilic polymer during or after formation of the fiber. Suitable superabsorbent materials can be selected from natural, synthetic, and modified natural polymers and materials. The superabsorbent materials can be inorganic materials, such as silica gels, or organic compounds, such as cross-linked polymers. In an embodiment, the absorbent body 59 can be free of superabsorbent material.

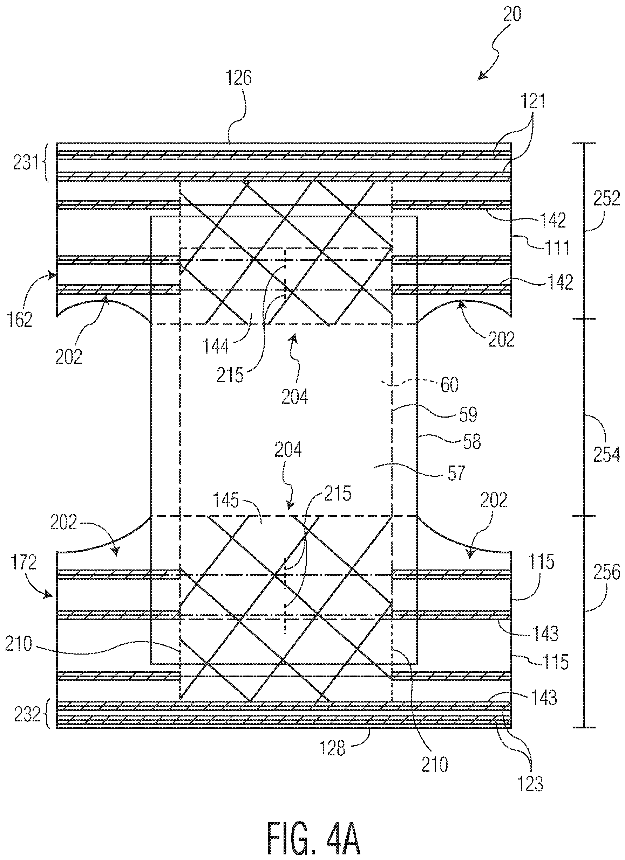

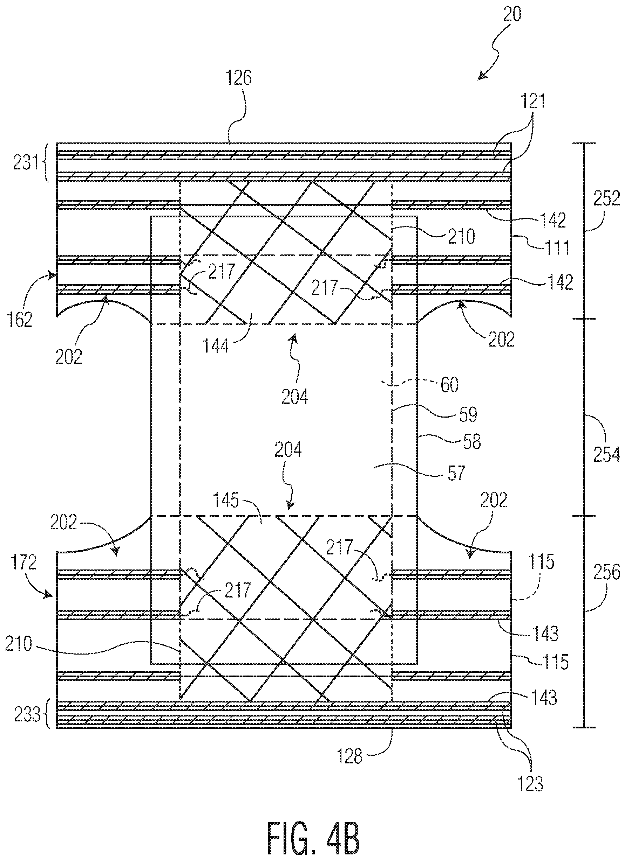

FIG. 3 further depicts front waistband region 231 and rear waistband region 232. As described previously, in some embodiments a portion of one of webs 111, 113 and/or 115, 117 may be folded over to encase one or more of the strands 121, 123 to form the waistband regions 231 and/or 232. In the embodiment of FIG. 3, the front waistband region 231 and the rear waistband region 232 are shown comprising two elastomeric strands 121, 123, respectively. However, in other embodiments the number of strands 121, 123 comprising the front waistband region 231 and/or the rear waistband region 232 may be any suitable number, such as between about 2 and about 15, or between about 4 and about 15, or between about 5 and about 10. In contrast to the elastomeric strands 121, 123 disposed outside of the waistband regions 231, 232, the elastomeric strands 121, 123 of the waistband regions 231, 232 may have had the first adhesives 143, 145 applied continuously, as can be better seen in FIGS. 4A and 4B.

Additionally, as can be seen, the absorbent insert 158 may span between the front waist panel 162 and the rear waist panel 172. FIGS. 4A and 4B further depict absorbent article 20 in an unfolded and laid flat configuration with the webs 111 and 117 removed to more clearly see the adhesives 142, 143, 144, 145 and their relation to the different components of the article 20, including the absorbent insert 58. As was the case in FIG. 2, the first adhesive 142 is shown disposed about the elastomeric strands 121 of the front waist panel 162. However, the first adhesive 142 is only disposed about the strands 121 in the first adhesive regions 202 and in the front waistband region 231. In the second adhesive region 204, there is no first adhesive 142 disposed about the strands 121. Rather, the second adhesive region 204 comprises the second adhesive 144. Likewise, in the rear waist panel 172 rear waist panel 172, the first adhesive 143 is shown disposed around the strands 123 in the first adhesive regions 202 and in the rear waistband region 232. The second adhesive 145 is shown disposed within the second adhesive region 204 of the rear waistband region 232. As described previously, this adhesive pattern may be achieved by intermittently applying the first adhesives 142, 143 to at least some of the elastomeric strands 121,123 and applying the second adhesives 143, 145 intermittently to the first front waist panel web 111, the second front waist panel web 113, or both, and to the first rear waist panel web 115, the second rear waist panel web 117, or both. The first adhesives 143, 145 have been applied continuously to the strands 121, 123 forming the front waistband region 231 and the rear waistband region 232.

Additionally, although the first adhesives 142, 143 are shown as having no overlap with the second adhesives 144, 145 in FIGS. 4A and 4B, in at least some embodiments, there may be regions where the adhesives 142, 143 do overlap the adhesives 144, 145, as was described with respect to FIG. 2. In the example of FIGS. 4A and 4B, the absorbent insert is shown spanning both the first adhesive regions 202 and the second adhesive regions 204 of both of the front waist panel 162 and the rear waist panel 172, while the absorbent body 59 only overlaps the second adhesive regions 204. In other embodiments, the whole absorbent insert 58 may only overlap the second adhesive regions 204 instead of both the first adhesive regions 202 and the second adhesive regions 204. Additionally, the absorbent insert 58 is shown as not overlapping the front waistband region 231 or the rear waistband region 232. However, this may not be the case in all embodiments.

FIG. 4A further calls out elastic cut points 215. In some embodiments, one or more of the elastomeric strands 121, 123 may be de-elasticized as described previously. In some embodiments where the strands 121, 123 cross the absorbent body 59, the strands 121, 123 may cause an undesirable bunching of the absorbent body 59, or other material within the vicinity of the absorbent body 59. One possible method for de-elasticizing one or more of the strands 121, 123 is to sever the strands 121, 123. The elastic cut points 215 demonstrate possible locations where the strands 121, 123 may be severed. Although each strand 121, 123 is only shown as having a single elastic cut point 215, in other embodiments, each of the strands 121, 123 may have multiple elastic cut points 215 at which the strands 121, 123 are severed. The elastic cut points 215 may generally be located within the second adhesive regions 204. In some embodiments, the second adhesives 144, 145 may not be sufficiently strong to withstand a retraction of the elastic strands 121, 123 when severed - as the strands 121, 123 were placed within the waist panels 162, 172 in a stretched state. FIG. 4B shows the article 20 with at least some of the strands 121, 123 severed, with the strand ends 217 called out.

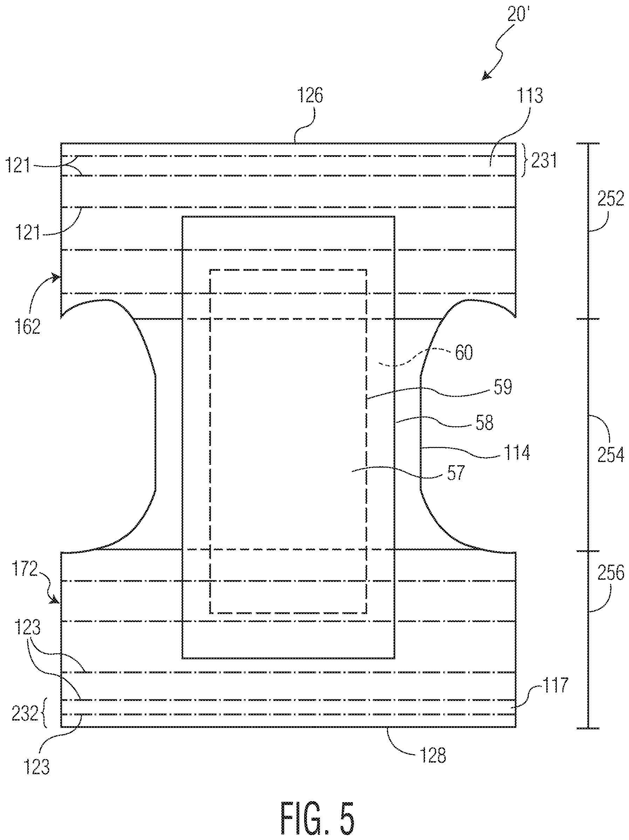

FIG. 5 depicts another exemplary absorbent article 20' in an unfolded and laid-flat configuration. The article 20' may differ from the article 20 in that the article 20' may have been made by the process 100B, using a unitary first waist panel material 114. Accordingly, article 20' of FIG. 5 has many of the same features as article 20 as shown in FIG. 3, but is notably missing the first front waist panel web 111 and the first rear waist panel web 115. Instead, the unitary first waist panel material 114 spans between the front waist region 252 and the rear waist region 256. The second front waist panel web 113 and the second rear waist panel web 117 are then both bonded to the unitary first waist panel material 114 with the strands 121, 123 disposed between the webs 113, 114, and 117.

## Claims

1. A method for assembling an absorbent article, the absorbent article comprising a chassis comprising a front waist region (252) having a front waist edge (126), a rear waist region (256) having a rear waist edge (128), and a crotch region (254) disposed between the front waist region (252) and the rear waist region (256), and an absorbent insert (58) disposed in the crotch region and coupled to the front waist region (252) and the rear waist region (256), the method comprising:
forming an elasticated first waist panel material comprising the steps of:
advancing a first continuous substrate material having an upper surface and a lower surface in a machine direction;
advancing a second continuous substrate material having an upper surface and a lower surface in the machine direction;
advancing a plurality of elastomeric strands (121) in a stretched state in the machine direction;
applying a first adhesive (142) intermittently to at least one of the plurality of elastomeric strands;
applying a second adhesive (144) intermittently to at least one of the lower surface of the first continuous substrate material and the upper surface of the second continuous substrate material, wherein the first adhesive (142) is different than the second adhesive (144);
placing the plurality of elastomeric strands (121) between the lower surface of the first continuous substrate material and the upper surface of the second continuous substrate material forming the elasticated first waist panel material such that:
the elasticated first waist panel material comprises first adhesive regions (202) comprising the first continuous substrate material, the second continuous substrate material, at least one of the plurality of elastomeric strands (121), and the intermittently applied first adhesive (142) but not the intermittently applied second adhesive (144), and
the elasticated first waist panel material comprises second adhesive regions (204) comprising the first continuous substrate material, the second continuous substrate material, at least one of the plurality of elastomeric strands (121), and the intermittently applied second adhesive (144) but not the intermittently applied first adhesive (142);
forming an elasticated second waist panel material;
coupling an absorbent insert (58) to the elasticated first waist panel material and the elasticated second waist panel material, the absorbent insert (58) comprising an absorbent body (59) and a bodyside liner (57); and
severing the elasticated first waist panel material and the elasticated second waist panel material to form an individual absorbent article (20), the elasticated first waist panel material forming the front waist region (252) and the elasticated second waist panel material forming the rear waist region (254).

2. The method of claim 1, further comprising bonding the elasticated first waist panel material to the elasticated second waist panel material.

3. The method of claim 1 or 2, further comprising applying the first adhesive (142) continuously to at least one of the plurality of elastomeric strands (121), and wherein the elasticated first waist panel material further comprises third adhesive regions comprising the first continuous substrate material, the second continuous substrate material, at least one of the plurality of elastomeric strands (121), and the continuously applied first adhesive (142) but not the second adhesive (144); optionally wherein the at least one of the plurality of elastomeric strands (121) having the first adhesive (142) continuously applied forms at least a portion of a front waistband (231) of the absorbent article (20) and is located closer to the front waist edge (126) of the absorbent article (20) than any of the plurality of elastomeric strands (121) having the first adhesive intermittently applied.

4. The method of any preceding claim, wherein the absorbent insert (58) is coupled to the elasticated first waist panel material such that the absorbent body (59) overlaps the second adhesive region (204) and none of the first adhesive regions (202); optionally wherein the absorbent insert (58) is coupled to the elasticated first waist panel material such that the absorbent body (59) overlaps the second adhesive region (204) but does not overlap the third adhesive region.

5. The method of any preceding claim, wherein the first adhesive (142) is an elastic adhesive; and/or wherein the second adhesive is a laminating adhesive (144).

6. The method of any preceding claim, wherein forming the elasticated second waist panel material comprises the steps of forming the elasticated first waist panel material.

7. An absorbent article (20) comprising:
a chassis comprising:
an elasticated front waist panel (162), the elasticated front waist panel comprising a front panel garment facing web (111), a front panel body facing web (113), and a plurality of elastomeric strands (121) disposed between the front panel garment facing web (111) and the front panel body facing web (113), the front waist panel (162) further comprising a first front panel adhesive region (202) comprising a first adhesive (142) but not a second adhesive (144) and a second front panel adhesive region (204) comprising the second adhesive (144) but not the first adhesive (142), wherein the first adhesive (142) is different than the second adhesive (144), and
an elasticated rear waist panel (172), the elasticated rear waist panel comprising a rear panel garment facing web (115), a rear panel body facing web (117), and a plurality of elastomeric strands (123) disposed between the rear panel garment facing web (115) and the rear panel body facing web (117), the rear waist panel (172) further comprising a first rear panel adhesive region (202) comprising the first adhesive (143) but not the second adhesive (145) and a second rear panel adhesive region (204) comprising the second adhesive (145) but not the first adhesive (143); and
an absorbent insert (58) coupled to the elasticated front waist panel (162) and the elasticated rear waist panel (172), the absorbent insert (58) comprising an absorbent body (59) and a body facing liner (57).

8. The absorbent article of claim 7, wherein a single web extends between the front waist panel (162) and the rear waist panel (172) forming the front panel garment facing web (111) and the rear panel garment facing web (115); and/or wherein the absorbent body (59) of the absorbent insert (58) overlaps the second front panel adhesive region (204) but not the first front panel adhesive region (202) and the second rear panel adhesive region (204) but not the first rear panel adhesive region (202).

## Patentansprüche

1. Verfahren zum Zusammenbauen eines absorbierenden Artikels, wobei der absorbierende Artikel ein Chassis umfasst, das einen vorderen Taillenbereich (252) mit einer vorderen Taillenkante (126), einen hinteren Taillenbereich (256) mit einer hinteren Taillenkante (128) und einem Schrittbereich (254), der zwischen dem vorderen Taillenbereich (252) und dem hinteren Taillenbereich (256) angeordnet ist, sowie eine absorbierende Einlage (58) umfasst, die in dem Schrittbereich angeordnet und mit dem vorderen Taillenbereich (252) und dem hinteren Taillenbereich (256) verbunden ist, das Verfahren umfassend:
Bilden eines elastischen ersten Tailleneinsatzmaterials, die folgenden Schritte umfassend:
Vorwärtsbewegen eines ersten kontinuierlichen Substratmaterials mit einer oberen Oberfläche und einer unteren Oberfläche in einer Maschinenrichtung;
Vorwärtsbewegen eines zweiten kontinuierlichen Substratmaterials mit einer oberen Oberfläche und einer unteren Oberfläche in der Maschinenrichtung;
Vorwärtsbewegen einer Vielzahl von Elastomersträngen (121) in einem gedehnten Zustand in der Maschinenrichtung;
intermittierendes Aufbringen eines ersten Klebstoffs (142) auf zumindest einen aus der Vielzahl von Elastomersträngen;
intermitterendes Aufbringen eines zweiten Klebstoffs (144) auf zumindest eine aus der unteren Oberfläche des ersten kontinuierlichen Substratmaterials und der oberen Oberfläche des zweiten kontinuierlichen Substratmaterials, wobei sich der erste Klebstoff (142) von dem zweiten Klebstoff (144) unterscheidet;
Platzieren der Vielzahl von Elastomersträngen (121) zwischen der unteren Oberfläche des ersten kontinuierlichen Substratmaterials und der oberen Oberfläche des zweiten kontinuierlichen Substratmaterials, wodurch das elastische erste Tailleneinsatzmaterial gebildet wird, sodass:
das elastische erste Tailleneinsatzmaterial erste klebende Bereiche (202) umfasst, die das erste kontinuierliche Substratmaterial, das zweite kontinuierliche Substratmaterial, zumindest einen aus der Vielzahl von Elastomersträngen (121) und den intermittierend aufgebrachten ersten Klebstoff (142), aber nicht den intermittierend aufgebrachten zweiten Klebstoff (144) umfassen, und
das elastische erste Tailleneinsatzmaterial zweite klebende Bereiche (204) umfasst, die das erste kontinuierliche Substratmaterial, das zweite kontinuierliche Substratmaterial, zumindest einen aus der Vielzahl von Elastomersträngen (121) und den intermittierend aufgebrachten zweiten Klebstoff (144), aber nicht den intermittierend aufgebrachten ersten Klebstoff (142) umfassen;
Bilden eines elastischen zweiten Tailleneinsatzmaterials;
Verbinden einer absorbierenden Einlage (58) mit dem elastischen ersten Tailleneinsatzmaterial und dem elastischen zweiten Tailleneinsatzmaterial, wobei die absorbierende Einlage (58) einen absorbierenden Körper (59) und eine körperseitige Auskleidung (57) umfasst; und
Durchtrennen des elastischen ersten Tailleneinsatzmaterials und des elastischen zweiten Tailleneinsatzmaterials, um einen einzelnen absorbierenden Artikel (20) zu bilden, wobei das elastische erste Tailleneinsatzmaterial den vorderen Taillenbereich (252) bildet und das elastische zweite Tailleneinsatzmaterial den hinteren Taillenbereich (254) bildet.

2. Verfahren nach Anspruch 1, ferner umfassend das Verkleben des elastischen ersten Tailleneinsatzmaterials mit dem elastischen zweiten Tailleneinsatzmaterial.

3. Verfahren nach Anspruch 1 oder 2, ferner das kontinuierliche Aufbringen des ersten Klebstoffs (142) auf mindestens einen aus der Vielzahl von Elastomersträngen (121) umfassend, und wobei das elastische erste Tailleneinsatzmaterial ferner dritte klebende Bereiche umfasst, die das erste kontinuierliche Substratmaterial, das zweite kontinuierliche Substratmaterial, mindestens einen aus der Vielzahl von Elastomersträngen (121), und den kontinuierlich aufgebrachten ersten Klebstoff (142), aber nicht den zweiten Klebstoff (144) umfassen; wobei der zumindest eine aus der Vielzahl von Elastomersträngen (121), bei dem der erste Klebstoff (142) kontinuierlich aufgebracht ist, optional zumindest einen Abschnitt eines vorderen Bunds (231) des absorbierenden Artikels (20) bildet und sich näher an der vorderen Taillenkante (126) des absorbierenden Artikels (20) befindet als irgendeiner aus der Vielzahl von Elastomersträngen (121), bei denen der erste Klebstoff intermittierend aufgebracht ist.

4. Verfahren nach einem der vorhergehenden Ansprüche, wobei die absorbierende Einlage (58) so mit dem elastischen ersten Tailleneinsatzmaterial verbunden ist, dass der absorbierende Körper (59) den zweiten klebenden Bereich (204) und keinen der ersten klebenden Bereiche (202) überlappt; wobei die absorbierende Einlage (58) optional so mit dem elastischen ersten Tailleneinsatzmaterial verbunden ist, dass der absorbierende Körper (59) den zweiten klebenden Bereich (204) überlappt, aber den dritten klebenden Bereich nicht überlappt.

5. Verfahren nach einem der vorhergehenden Ansprüche, wobei der erste Klebstoff (142) ein elastischer Klebstoff ist; und/oder wobei der zweite Klebstoff ein Laminierklebstoff (144) ist.

6. Verfahren nach einem der vorhergehenden Ansprüche, wobei das Bilden des elastischen zweiten Tailleneinsatzmaterials die Schritte des Bildens des elastischen ersten Tailleneinsatzmaterials umfasst.

7. Absorbierender Artikel (20), umfassend:
ein Chassis, umfassend:
einen elastischen vorderen Tailleneinsatz (162), wobei der elastische vordere Tailleneinsatz eine zur Kleidung ausgerichtete Bahn des vorderen Einsatzes (111), eine körperseitige Bahn des vorderen Einsatzes (113) und eine Vielzahl von Elastomersträngen (121) umfasst, die zwischen der zur Kleidung ausgerichteten Bahn des vorderen Einsatzes (111) und der körperseitigen Bahn des vorderen Einsatzes (113) angeordnet sind, wobei der vordere Tailleneinsatz (162) ferner einen klebenden Bereich des ersten vorderen Einsatzes (202), der einen ersten Klebstoff (142), aber keinen zweiten Klebstoff (144) umfasst, und einen zweiten klebenden Bereich des vorderen Einsatzes (204) umfasst, der den zweiten Klebstoff (144), aber nicht den ersten Klebstoff (142) umfasst, wobei der erste Klebstoff (142) sich von dem zweiten Klebstoff (144) unterscheidet, und
einen elastischen hinteren Tailleneinsatz (172), wobei der elastische hintere Tailleneinsatz eine zur Kleidung ausgerichtete Bahn des hinteren Einsatzes (115), eine körperseitige Bahn des hinteren Einsatzes (117) und eine Vielzahl von Elastomersträngen (123) umfasst, die zwischen der zur Kleidung ausgerichteten Bahn des hinteren Einsatzes (115) und der körperseitigen Bahn des hinteren Einsatzes (117) angeordnet sind, wobei der hintere Tailleneinsatz (172) ferner einen klebenden Bereich des ersten hinteren Einsatzes (202), der den ersten Klebstoff (143), aber nicht den zweiten Klebstoff (145) umfasst, und einen zweiten klebenden Bereich des hinteren Einsatzes (204) umfasst, der den zweiten Klebstoff (145), aber nicht den ersten Klebstoff (143) umfasst; und
eine absorbierende Einlage (58), die mit dem elastischen vorderen Tailleneinsatzmaterial (162) und dem elastischen hinteren Tailleneinsatzmaterial (172) verbunden ist, wobei die absorbierende Einlage (58) einen absorbierenden Körper (59) und eine körperseitige Auskleidung (57) umfasst.

8. Absorbierender Artikel nach Anspruch 7, wobei sich eine einzelne Bahn zwischen dem vorderen Tailleneinsatz (162) und dem hinteren Tailleneinsatz (172) erstreckt und die zur Kleidung ausgerichtete Bahn des vorderen Einsatzes (111) und die zur Kleidung ausgerichtete Bahn des hinteren Einsatzes (115) bildet; und/oder wobei der absorbierende Körper (59) der absorbierenden Einlage (58) den zweiten klebenden Bereich des vorderen Einsatzes (204), aber nicht den ersten klebenden Bereich des vorderen Einsatzes (202), und den zweiten klebenden Bereich des hinteren Einsatzes (204), aber nicht den ersten klebenden Bereich des hinteren Einsatzes (202) überlappt.

## Revendications

1. Procédé d'assemblage d'un article absorbant, l'article absorbant comprenant une structure comprenant une région de taille avant (252) ayant un bord de taille avant (126), une région de taille arrière (256) ayant un bord de taille arrière (128), et une région d'entrejambe (254) disposée entre la région de taille avant (252) et la région de taille arrière (256), et une garniture absorbante (58) disposée dans la région d'entrejambe et couplée à la région de taille avant (252) et la région de taille arrière (256), le procédé comprenant :
la formation d'un premier matériau élastique de panneau de taille comprenant les étapes consistant à :
faire avancer un premier matériau de substrat continu ayant une surface supérieure et une surface inférieure dans un sens machine ;
faire avancer un deuxième matériau de substrat continu ayant une surface supérieure et une surface inférieure dans le sens machine ;
faire avancer une pluralité de brins élastomères (121) dans un état étiré dans le sens machine ;
appliquer un premier adhésif (142) par intermittence à l'au moins un de la pluralité de brins élastomères ;
appliquer un deuxième adhésif (144) par intermittence à l'au moins une parmi la surface inférieure du premier matériau de substrat continu et la surface supérieure du deuxième matériau de substrat continu, dans lequel le premier adhésif (142) est différent du deuxième adhésif (144) ;
le placement de la pluralité de brins élastomères (121) entre la surface inférieure du premier matériau de substrat continu et la surface supérieure du deuxième matériau de substrat continu formant le premier matériau élastique de panneau de taille de telle sorte que :
le premier matériau élastique de panneau de taille comprend des premières régions adhésives (202) comprenant le premier matériau de substrat continu, le deuxième matériau de substrat continu, au moins l'un de la pluralité de brins élastomères (121), et le premier adhésif appliqué de manière intermittente (142) mais pas le deuxième adhésif appliqué de manière intermittente (144), et
le premier matériau élastique de panneau de taille comprend des deuxièmes régions adhésives (204) comprenant le premier matériau de substrat continu, le deuxième matériau de substrat continu, au moins l'un de la pluralité de brins élastomères (121), et le deuxième adhésif appliqué de manière intermittente (144) mais pas le premier adhésif appliqué de manière intermittente (142) ;
former un deuxième matériau élastique de panneau de taille ;
coupler une garniture absorbante (58) au premier matériau élastique de panneau de taille et au deuxième matériau élastique de panneau de taille, la garniture absorbante (58) comprenant un corps absorbant (59) et une doublure côté corps (57) ; et
séparer le premier matériau élastique de panneau de taille et le deuxième matériau élastique de panneau de taille pour former un article absorbant individuel (20), le premier matériau élastique de panneau de taille formant la région de taille avant (252) et le deuxième matériau élastique de panneau de taille formant la région de taille arrière (254).

2. Procédé selon la revendication 1, comprenant en outre la liaison du premier matériau élastique de panneau de taille au deuxième matériau élastique de panneau de taille.

3. Procédé selon la revendication 1 ou 2, comprenant en outre l'application du premier adhésif (142) de manière continue à au moins l'un de la pluralité de brins élastomères (121), et dans lequel le premier matériau élastique de panneau de taille comprend en outre des troisièmes régions adhésives comprenant le premier matériau de substrat continu, le deuxième matériau de substrat continu, au moins l'un de la pluralité de brins élastomères (121), et le premier adhésif appliqué en continu (142) mais pas le deuxième adhésif (144) ; facultativement dans lequel l'au moins un parmi la pluralité de brins élastomères (121) ayant le premier adhésif (142) appliqué en continu forme au moins une partie d'une ceinture avant (231) de l'article absorbant (20) et est situé plus près du bord de taille avant (126) de l'article absorbant (20) que l'un quelconque de la pluralité de brins élastomères (121) ayant le premier adhésif appliqué de manière intermittente.

4. Procédé selon l'une quelconque des revendications précédentes, dans lequel la garniture absorbante (58) est couplée au matériau élastique du premier panneau de taille de telle sorte que le corps absorbant (59) chevauche la deuxième région adhésive (204) et aucune des premières régions adhésives (202) ; facultativement dans lequel la garniture absorbante (58) est couplée au matériau élastique de premier panneau de taille de telle sorte que le corps absorbant (59) chevauche la deuxième région adhésive (204) mais ne chevauche pas la troisième région adhésive.

5. Procédé selon l'une quelconque des revendications précédentes, dans lequel le premier adhésif (142) est un adhésif élastique ; et/ou dans lequel le deuxième adhésif est un adhésif de stratification (144).

6. Procédé selon l'une quelconque des revendications précédentes, dans lequel la formation du deuxième matériau élastique de panneau de taille comprend les étapes consistant à former le premier matériau élastique de panneau de taille.

7. Article absorbant (20) comprenant :
une structure comprenant :
un panneau élastique de taille avant (162), le panneau élastique de taille avant comprenant une bande faisant face au vêtement de panneau avant (111), une bande faisant face au corps de panneau avant (113), et une pluralité de brins élastomères (121) disposés entre la bande faisant face au vêtement de panneau avant (111) et la bande faisant face au corps de panneau avant (113), le panneau de taille avant (162) comprenant en outre une première région adhésive de panneau avant (202) comprenant un premier adhésif (142) mais pas un deuxième adhésif (144) et une deuxième région adhésive de panneau avant (204) comprenant le deuxième adhésif (144) mais pas le premier adhésif (142), dans lequel le premier adhésif (142) est différent du deuxième adhésif (144), et
un panneau élastique de taille arrière (172), le panneau élastique de taille arrière comprenant une bande faisant face au vêtement du panneau arrière (115), une bande faisant face au corps de panneau arrière (117), et une pluralité de brins élastomères (123) disposés entre la bande faisant face au vêtement de panneau arrière (115) et la bande faisant face au corps de panneau arrière (117), le panneau de taille arrière (172) comprenant en outre une première région adhésive de panneau arrière (202) comprenant le premier adhésif (143) mais pas le deuxième adhésif (145) et une deuxième région adhésive de panneau arrière (204) comprenant le deuxième adhésif (145) mais pas le premier adhésif (143) ; et
une garniture absorbante (58) couplée au panneau élastique de taille avant (162) et au panneau élastique de taille arrière (172), la garniture absorbante (58) comprenant un corps absorbant (59) et une doublure faisant face au corps (57).

8. Article absorbant selon la revendication 7, dans lequel une unique bande s'étend entre le panneau de taille avant (162) et le panneau de taille arrière (172) formant la bande faisant face au vêtement de panneau avant (111) et la bande faisant face au vêtement de panneau arrière (115) ; et/ou dans lequel le corps absorbant (59) de la garniture absorbante (58) chevauche la deuxième région adhésive de panneau avant (204) mais pas la première région adhésive de panneau avant (202) et la deuxième région adhésive de panneau arrière (204) mais pas la région adhésive de panneau arrière (202).
